# EUROPEAN PATENT APPLICATION

(11) **EP 4 502 164 A1**
(43) Date of publication of application: **05.02.2025**
(21) Application number: 23777819.6
(22) Date of filing: 14.03.2023
(51) Int. Cl.: C12N 15/85, C12N 15/90, C12N 15/11, A61K 48/00, A61P 3/10

(54) **GLUCOSE-REGULATED GENE EXPRESSION LOOP CONTROL SYSTEM AND USE THEREOF IN REGULATING AND CONTROLLING BLOOD GLUCOSE**

(30) Priority: 30.03.2022 CN 202210326236
(71) Applicant: East China Normal University, Shanghai 200241 (CN)
(72) Inventor: YE, Haifeng, Shanghai 200241 (CN); GUAN, Ningzi, Shanghai 200241 (CN); GAO, Xianyun, Shanghai 200241 (CN)
(74) Representative: karo IP
(86) International application number: PCT/CN2023/081232
(87) International publication number: WO 2023/185442

(57) **Abstract**

Provided are a glucose-regulated gene expression loop control system and use thereof in regulating and controlling blood glucose. The control system comprises a transcription repressor HexR, a glucose-induced strong promoter, and a sequence to be transcribed. Also provided are an expression vector, an engineered cell, a sensor, or a recombinant probiotic comprising the control system and use thereof in preparing a drug for treating diabetes. Further provided are a plasmid, a glucose-induced strong promoter, and a primer pair which are involved in the control system.

## Description

### BACKGROUND

### Technical Field

The present invention relates to the fields of synthetic biology and cell therapy, combining microbial synthetic biology with disease treatment, specifically a glucose-regulated gene expression loop control system and use thereof in regulating and controlling blood glucose.

### Related Art

Biosensors can directly use complete microbial cells or cell fragments as sensitive materials, utilizing their internal enzymes and metabolic systems to recognize and detect various substances. The development of synthetic biology in recent years has greatly promoted the functionality of biosensors. In biomedical fields, they can effectively detect various physiological indicators or disease signals, including stimuli and external signals in the host environment. Probiotics can specifically target the intestine as an intestinal-targeted drug delivery carriers, meeting almost all requirements as biological therapeutic agents. Developing biosensors using probiotics to achieve diagnosis and treatment integration is an important means to achieve precision medicine.

Currently, diabetes treatments mainly include insulin injections, medication, and diet control. However, the current medical technology still cannot completely cure diabetes. Diabetic patients need to take hypoglycemic drugs orally or inject insulin daily to maintain stable blood glucose levels. Using oral or injection methods, most of the active ingredients in current clinical drugs will become inactive, and only a small part can act on the intended target site. If the drug's efficiency is insufficient or too low, the required drug dose increases significantly, often harming the patient's health and causing numerous side effects. Additionally, insulin injections cannot achieve controlled insulin release, easily leading to hypoglycemia risks. The ability to automatically monitor the blood glucose concentration in the body in real time and accurately adjust the blood glucose concentration in time is very important for the treatment of diabetes. Therefore, it is crucial to develop new treatment methods to improve treatment efficacy, reduce treatment risks, and enhance convenience of treatment.

On the other hand, impaired wound healing process is an increasingly serious medical problem closely related to metabolic diseases and aging. Open wounds on the skin can cause significant discomfort and provide an entry point for bacterial invasion. During the inflammatory phase of wound healing, immune cells accumulate under the influence of alarm signals, cytokines, and chemokines released by injured or activated cells, thus playing a crucial role in wound healing. Macrophages and neutrophils are the main immune cell groups at the injury site. While preventing microbial invasion, they can also promote the healing process by secreting additional chemokines, growth factors, and matrix digestion enzymes.

Chronic wounds are often associated with underlying pathological processes that can increase wound susceptibility or reduce healing ability, such as arterial/venous insufficiency, diabetes, or undergoing systemic steroid therapy. For example, diabetic foot ulcer, as a common complication of diabetes, causes prolonged pain, reduced vitality, and may even require amputation. Standard treatments for chronic wounds like diabetic foot ulcers include surgical or chemical removal of necrotic tissue, repeated dressing changes, and using antibiotics to control infection. The results are often unstable and accompanied by adverse side effects. Additionally, clinical treatments using local application of growth factors or combined with different biomaterials for various chronic wounds, but the effect is not significant. Wherein, the proteolytic characteristics of wounds limits the utilization of drugs, posing a major obstacle to chronic wound treatment. Therefore, new treatment methods are urgently needed for stable and safe treatment of difficult-to-heal chronic skin wounds, improving treatment efficacy and convenience.

### SUMMARY

The present invention proposes a glucose-regulated gene expression loop control system comprising a transcription repressor HexR, a glucose-induced strong promoter, and a sequence to be transcribed. The transcription repressor HexR can undergo homodimerization and bind to specific DNA sequences selected from SEQ ID NO.78-80. The glucose-induced strong promoter is composed of a constitutive strong promoter combined with the specific DNA sequence, including but not limited to any nucleotide sequence from SEQ ID NO.1-10.

Wherein, the transcription repressor can bind to the glucose-induced strong promoter and thereby inhibit the expression of downstream sequences to be transcribed. The sequence to be transcribed includes a single coding expression reporter protein or functional protein, or a tandem expression of multiple proteins. The transcription repressor, glucose-induced strong promoter, and a sequence to be transcribed can be constructed on one or two plasmid vectors.

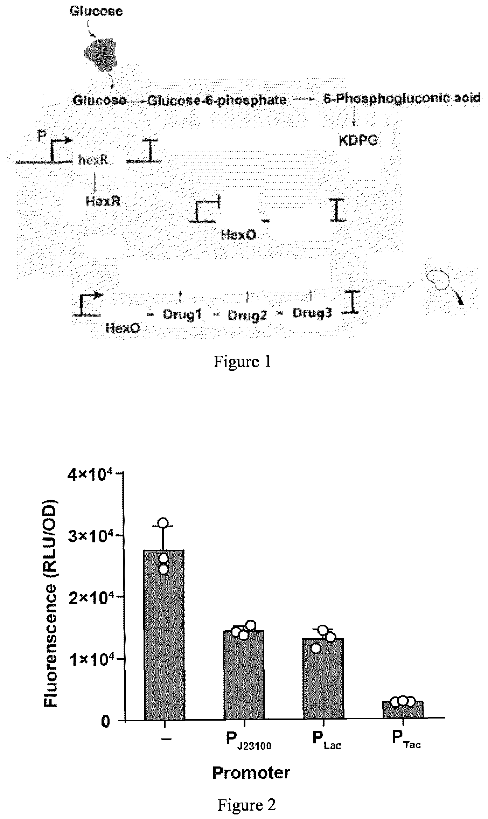

The present invention also proposes a glucose-induced gene expression regulation method, which is regulated by the glucose-regulated gene expression loop control system. In specific embodiments, when glucose is absent, the transcription repressor can bind to the glucose-induced strong promoter, thereby inhibiting the expression of downstream sequences to be transcribed; when glucose is present, glucose is metabolized through the Entner-Doudoroff pathway to produce the metabolic intermediate 2-keto-3-deoxy-6-phosphogluconate (KDPG), which blocks binding between the transcription repressor and the glucose-induced strong promoter, causing the transcription repressor to dissociate from the glucose-induced strong promoter and initiate the expression of the downstream sequence to be transcribed. In specific embodiments, the regulation method of inducing gene expression is shown in Figure 1.

In the present invention, the glucose-regulated gene expression loop control system, namely, glucose precisely regulates the gene initiation expression and secretion, comprising three parts: the transcription repressor HexR, the glucose-induced strong promoter, and the sequence to be transcribed.

Wherein, the transcription repressor HexR is a repressor protein HexR of the HexR operon system derived from Pseudomonas putida (amino acid sequence Genbank accession number: AE015451), continuously expressed by a strong promoter.

Preferably, the transcription repressor HexR can be initiated expression by various types of strong promoters, including but not limited to PBBA23100 (nucleotide sequence Genbank accession number: MG649435), PLac (nucleotide sequence Genbank accession number: LC652750), PTac (nucleotide sequence Genbank accession number: MN913428), etc.

Wherein, the glucose-induced strong promoter is constructed by inserting different copies of the HexR operon binding sites between the strong promoter and the RBS site. The HexR operon binding sites are derived from the operon element HexO of the HexR operon system. The glucose-induced strong promoter can drive the expression of downstream genes. The HexR operon system consists of the repressor protein HexR and the operon element HexO. It was originally isolated from Pseudomonas putida and is strictly regulated by the glucose metabolite 2-keto-3-deoxy-6-phosphogluconate (KDPG).

Preferably, the glucose-induced strong promoters can be constructed according to the type of strong promoter and the different copy numbers of the HexO operon, forming different types of hybrid strong promoters, including:
a) The glucose-induced strong promoter PHexR1 with nucleotide sequence PHexR1 (PTac-HexO) as shown in SEQ ID NO.1.
b) The glucose-induced strong promoter PHexR2 with nucleotide sequence PHexR2 (PTac-2×HexO) as shown in SEQ ID NO.2.
c) The glucose-induced strong promoter PHexR3 with nucleotide sequence PHexR3 (PTac-3×HexO) as shown in SEQ ID NO.3.
d) The glucose-induced strong promoter PHexR4 with nucleotide sequence PHexR4 (PTac-4×HexO) as shown in SEQ ID NO.4.
e) The glucose-induced strong promoter PHexRS with nucleotide sequence PHexR5 (PTac-5×HexO) as shown in SEQ ID NO.5.
f) The glucose-induced strong promoter PHexR6 with nucleotide sequence PHexR6 (PLac-HexO) as shown in SEQ ID NO.6.
g) The glucose-induced strong promoter PHexR7 with nucleotide sequence PHexR7 (PLac-2×HexO) as shown in SEQ ID NO.7.
h) The glucose-induced strong promoter PHexR8 with nucleotide sequence PHexR8 (PLac-3×HexO) as shown in SEQ ID NO.8.
i) The glucose-induced strong promoter PHexR9 with nucleotide sequence PHexR9 (PLac-4×HexO) as shown in SEQ ID NO.9.
j) The glucose-induced strong promoter PHexR10 with nucleotide sequence PHexRlO (PLac-5×HexO) as shown in SEQ ID NO.10, and a glucose-induced promoters PHexRn constructed from any constitutive promoter and any number of HexO copies.

Wherein, the sequence to be transcribed can be a single coding expression reporter protein such as sfGFP (amino acid sequence Genbank accession number: AB971579), LuxCDABE (amino acid sequence Genbank accession number: EF173694), or a functional protein such as the glucagon-like peptide GLP-1 (amino acid sequence shown in SEQ ID NO.11), the glucagon-like peptide EK-GLP-1 (amino acid sequence shown in SEQ ID NO.12), the glucagon-like peptide GLP-1-Fc (amino acid sequence shown in SEQ ID NO.13), the mouse pancreatic duodenal homeobox-1 (mPDX-1) (amino acid sequence shown in SEQ ID NO.61), or the human pancreatic duodenal homeobox-1 (hPDX-1) (amino acid sequence shown in SEQ ID NO.66).

Wherein, the glucose-regulated gene expression loop control system can precisely regulate the expression of one or multiple reporter proteins and functional proteins in tandem via polycistronic operons.

Wherein, the glucose-regulated gene expression loop control system is implemented using an artificially designed and synthesized dual-plasmid system. The sequences involved in the dual-plasmid system are detailed in Table 1.

The present invention also provides an expression vector, an engineered cell, a sensor, or a recombinant probiotic containing the described glucose-regulated gene expression loop control system. In specific embodiments, the glucose-regulated gene expression loop control system is located in any of the chromosome or plasmid of the engineered cells, sensors, or recombinant probiotics.

Additionally, the present invention proposes an application, namely, the glucose-regulated gene expression loop control system, the expression vector, engineered cells, sensors, or recombinant probiotics in the preparation of drugs for the treatment and/or prevention of diabetes and the drugs or products of the regulation of blood glucose levels.

In specific embodiments, the drugs or products include but are not limited to various formulations such as oral agents, topical agents, injections, etc. Preferably, the oral agent method is adopted. In specific embodiments, by orally administering recombinant probiotics containing the glucose-regulated gene expression loop control system (i.e., intelligent controllable microbial drug factories), induces gene expression regulation by glucose, thereby expressing hypoglycemic peptides to regulate blood glucose. In other words, by orally administering the recombinant probiotics (intelligent and controllable probiotic drug factories that have been transformed), it can be achieved by the method of inducing glucagon-like-peptide by blood glucose, which achieves precise regulation of GLP-1 or/and PDX-1 expression, thereby achieving the effect of treating diabetes. Specifically, in the application and treatment methods, blood glucose levels are regulated by controlling the release of glucagon-like peptide and pancreatic duodenal homeobox-1 hypoglycemic protein.

The present invention also proposes a method for regulating blood glucose, which utilizes the glucose-regulated gene expression loop control system and/or the expression vector, engineered cells, sensors, or recombinant probiotics to regulate blood glucose. In specific embodiments, the method achieves the effect of regulating blood glucose by controlling the expression of GLP-1 and PDX-1 hypoglycemic proteins. Specifically, the method for regulating blood glucose includes the following steps:
a) Artificially constructing a prokaryotic expression vector or functional module for chromosomal integration; the prokaryotic expression vector or functional module for chromosomal integration contains a glucose-induced GLP-1/PDX-1 expression regulation system;
b) Transforming the expression vector or functional module for chromosomal integration constructed in step a) into microbial cells or integrating it into the microbial cell chromosome to prepare engineered cells containing the glucose-induced GLP-1/PDX-1 expression regulation system;
c) Administering the engineered cells prepared in step b) via oral gavage into diabetic model mice;
d) Glucose closed-loop induction in mice induces the engineered cells to express and secrete GLP-1/PDX-1, which is absorbed into the bloodstream to lower blood glucose.

In specific embodiments, the method for regulating blood glucose can be administered by oral probiotics.

The present invention also provides a novel method for treating diabetes. By using the intelligent controllable probiotic drug factories and the glucose-induced GLP-1 and/or PDX-1 expression regulation system, orally administrating probiotics can achieve the effect of lowering blood glucose. The present invention offers a safe, reliable, and precisely controlled new strategy for the release of glucagon-like peptide and/or pancreatic duodenal homeobox-1 for treating diabetes. The present invention presents a new method and strategy for treating diabetes. The system can regulate the expression of glucagon-like peptide (GLP-1) and pancreatic duodenal homeobox-1 (PDX-1). The expression of glucagon-like peptide (GLP-1)includes EK-GLP-1, GLP-1-Fc, etc. The expression of pancreatic duodenal homeobox-1 (PDX-1) includes mPDX-1, hPDX-1, etc. The glucose-regulated gene expression loop control system of the present invention can rapidly regulate gene expression through glucose metabolites, characterized by precise control of gene expression levels and high multiplicity of gene regulation.

The glucose-regulated gene expression loop control system of the present invention exhibits fine-tuning and reversible expression kinetics characteristics. The fine-tuning refers to the precise regulation of downstream gene expression by glucose, showing a dose-dependent relationship. The reversibility means that the entire process of glucose-regulated gene expression is reversible and can be turned on or off by controlling the presence or absence of glucose. These fine-tuning and reversible expression kinetics characteristics are also reflected in the glucose-induced gene expression regulation method, blood glucose regulation method, and in the expression vectors, engineered cells, sensors, or recombinant probiotics.

The present invention also proposes a plasmid, which is selected from one or more of pGN11, pGN69, pGN89, pGN90, pGN227, pGN12, pGN228, pGN229, pGN220, pGN221, pGN222, pGN223, pGN224, pGN231, pGN232, pGN233, pGN65, pGN237, pGN238, pGN239, pGN241, pGN242, pGN243, pGN288, pGN308, pGN306, pGN307, and pXG58. The plasmids are detailed in Table 1.

The present invention also proposes a nucleotide sequence selected from glucose-induced strong promoters PHexR1, PHexR2, PHexR3, PHexR4, PHexR5, PHexR6, PHexR7, PHexR8, PHexR9, PHexR10, whose nucleotide sequences are respectively shown in SEQ ID NO.1 to SEQ ID NO.10.

The present invention also proposes a primer, whose sequences are one or more of the sequences shown in SEQ ID NO.16-60, 62-65, 67-74, 76-77. The present invention further proposes a primer pair, whose sequences are one or more of the sequences shown in Primer Pairs 1-30. These sequences are detailed in Table 1 and Table 2.

The present invention also proposes an intelligent controllable microbial drug factory, which includes a chassis microorganism and a gene expression loop control system loaded onto the chassis microorganism. In specific embodiments, the gene expression loop control system is located on either the chromosome or the plasmid of the chassis microorganism.

The intelligent controllable microbial drug factory of the present invention is preferably a glucose-regulated probiotic drug factory, which is engineered cells contain the glucose-regulated gene expression loop control system. It can also be a microbial drug factory regulated by other means, namely, engineered cells with other types of gene expression loop control systems. In specific embodiments, the engineered cells can be any probiotic, such as Escherichia coli Nissle 1917 (EcN), etc.

Wherein, the gene expression loop control system includes, but is not limited to, a glucose-regulated gene expression loop control system and other regulation methods such as xylose-induced gene expression systems, arabinose-induced gene expression systems, uric acid-induced gene expression systems, etc.

In the present invention, the glucose-regulated gene expression loop control system includes three parts: the transcription repressor HexR, the glucose-induced strong promoter, and the sequence to be transcribed. In a specific embodiment, the transcription repressor HexR can bind to the glucose-induced strong promoter and thereby repress the expression of the downstream sequence to be transcribed. The glucose-induced strong promoter is composed of a constitutive strong promoter and a DNA sequence specifically binding to HexR. The sequence to be transcribed includes single coding sequences expressing reporter proteins such as LuxCDABE (amino acid sequence Genbank accession number: EF173694) or functional proteins such as human acidic fibroblast growth factor rhaFGF135 amino acid sequence shown in SEQ ID NO. 14, chemokine CXCL12 amino acid sequence shown in SEQ ID NO. 15, human interleukin-4 (hIL4) amino acid sequence shown in SEQ ID NO.75, or tandem expression of multiple proteins. In specific embodiments, the regulation method is as follows: when glucose is absent, the transcription repressor can bind to the glucose-induced strong promoter and thereby repress the expression of the downstream sequence to be transcribed. When glucose is present, it is metabolized via the Entner-Doudoroff pathway to generate the metabolic intermediate 2-keto-3-deoxy-6-phosphogluconate (KDPG). KDPG blocks the binding of HexR, causing the transcription repressor to dissociate from the glucose-induced strong promoter, thus initiating the expression of the downstream target transcription sequence.

In the present invention, the gene expression loop control system includes a transcription repressor, an inducible strong promoter, and a sequence to be transcribed. In specific embodiments, the transcription repressor, inducible strong promoter, and sequence to be transcribed can be constructed on one or two plasmid vectors.

In the present invention, the intelligent controllable microbial drug factory can express the target gene in the presence of an inducer. In specific embodiments, the inducer can be externally added or produced in vivo.

Preferably, the chassis microorganisms include probiotics. Preferably, the probiotics include, but are not limited to, Escherichia coli Nissle 1917, Lactococcus lactis, Lactobacillus plantarum, Bacillus subtilis, etc. In specific embodiments, the intelligent controllable microbial drug factory is recombinant probiotics.

The intelligent controllable microbial drug factory of the present invention includes but is not limited to be in liquid form or in lyophilized or spray-dried form.

The present invention also proposes a method for constructing the intelligent controllable microbial drug factory, including the following steps:
a) Combining a DNA operon sequence specifically recognized by a transcription repressor protein with a constitutive strong promoter to construct an inducible strong promoter;
b) Constructing the coding gene for the therapeutic protein downstream of the inducible promoter, either singly or in tandem, to create a reporter module. The type and number of therapeutic proteins can be replaced or changed as needed;
c) Constructing a functional module that constitutively expresses the transcription repressor protein.

The method for constructing an intelligent controllable microbial drug factory peoposed by the present invention involves transforming the plasmid containing the functional module and reporting module constructed in the steps b) and c) into competent probiotic cells, or integrating the functional module and reporting module constructed in the steps b) and c) into the probiotic chromosome through the RED recombinase system or CRISPR system, to prepare inducible engineered cells, namely, to prepare the intelligent controllable microbial drug factory.

The present invention also proposes expression vectors and engineered cells, containing the intelligent controllable microbial drug factory. In specific embodiments, it provides a prokaryotic expression vector containing the glucose-regulated gene expression loop control system.

The present invention also proposes the application of the intelligent controllable microbial drug factory in the preparation of drugs or products for the prevention and/or treatment of diseases. The drugs or products include but are not limited to drugs that promote skin wound healing, drugs that prevent and/or treat metabolic diseases, and drugs that prevent and/or treat other diseases, etc.

In specific embodiments, the skin wounds include, but are not limited to, chronic skin wounds, diabetic skin wounds, and diabetic foot ulcers, etc.

The present invention also proposes the application of an intelligent controllable microbial (preferably probiotic) drug factory in promoting the healing of diabetic skin wounds. The application for promoting diabetic skin wound healing is achieved by the in-situ application of the intelligent controllable probiotic drug factory to the wound,which is done by constructing an intelligent controllable probiotic drug factory that is induced by blood glucose to produce growth factors, cytokines, and chemokines, namely, the treatment method utilizes the intelligent controllable probiotic drug factory to precisely regulate the expression and secretion of growth factors, cytokines, and chemokines to promote wound healing.

The present invention further proposes a method for promoting skin wound healing using the intelligent controllable microbial (probiotic) drug factory. The method includes the following steps:
a) Artificially construct a prokaryotic expression vector for expression regulation system, which contains glucose-induced growth factors, macrophage M2 polarization cytokines and chemokines, or a functional module for chromosomal integration;
b) Prepare engineered cells containing glucose-induced growth factors, macrophage M2 polarization cytokines and chemokine expression regulation system;
c) Transplant the engineered cells prepared in the step b) to the skin wounds of diabetic model mice in the form of in-situ application to the wounds;
d) Glucose closed-loop induction at the mouse skin wounds induces the engineered cells to express and secrete growth factors, macrophage M2 polarization cytokines, and chemokines, to promote wound healing.

Wherein, the growth factors are rhaFGF135, FGF2, FGF7, TGF-α, TGF-β, PDGF, EGF, VEGF, IGF-1, IGF-2, PDGF, HGF; the macrophage M2 polarization cytokines are IL-4, IL-10, IL-13, CSF1, IL34; the chemokines are CXCL12, CXCL13, CXCL8, CCL2, CCL3, CCL4, CCL5, CCL11, CXCL10.

The present invention also proposes a plasmid, the plasmid is one or several of plasmids pGN233, pGN65, pGN299, pGN300, pGN314, as detailed in Table 1.

The present invention further proposes a nucleotide sequence, which is one of the glucose-induced strong promoters PHexR4 or PHexR9, with sequences shown as SEQ ID NO.4 and SEQ ID NO.9, respectively.

The beneficial effects of the present invention include providing a new method for promoting the healing of chronic skin wounds. Through the intelligent controllable probiotic drug factory, glucose-induced growth factors, cytokines, and chemokine expression regulation system, using the method of applying probiotics in situ to the wound can achieve the effect of accelerating wound healing. The present invention offers a safe, reliable, and precisely controllable new strategy for accelerating the healing of chronic skin wounds and diabetic foot ulcers. The system can regulate the expression and secretion of growth factors, macrophage M2 polarization cytokines and chemokines. The present invention innovatively develops an optimized technology for directly delivering growth factors, macrophage M2 polarization cytokines, and chemokines to injured skin using probiotics as carriers. Plasmids or chromosomal genomes encode the growth factors, macrophage M2 polarization cytokines, and chemokines.

The beneficial effects of the present invention include providing an intelligent controllable probiotic drug factory, which includes a gene expression loop control system and chassis probiotics. The glucose-regulated gene expression loop control system of the present invention exhibits fine-tuning and reversible expression kinetics characteristics. The present invention also provides the application of the intelligent controllable probiotic drug factory, the prokaryotic expression vector containing the glucose-regulated gene expression loop control system, or the engineered cells containing the glucose-regulated gene expression loop control system in the treatment of diabetes, promoting the healing of chronic skin wounds, promoting the healing of diabetic foot ulcers and other chronic skin wounds, and the prevention and/or treatment of metabolic diseases.

### BRIEF DESCRIPTION OF THE DRAWINGS

Figure 1 shows the schematic diagram of the principle of the glucose-regulated gene expression loop control system and regulation method of the present invention.
Figure 2 shows the optimization study of the glucose-regulated gene expression loop control system of the present invention, namely, the experimental results of combining different promoters driving the expression of HexR with the glucose-induced strong promoter PHexR1.
Figure 3 shows the optimization study of the glucose-regulated gene expression loop control system of the present invention, namely, the experimental results of combining the PTac promoter-driven expression of HexR with five different response elements.
Figure 4 shows the optimization study of the glucose-regulated gene expression loop control system of the present invention, namely, the experimental results of combining RBS-regulated HexR expression with the glucose-induced strong promoter PHexR4.
Figure 5 shows the experimental results demonstrating the tunable expression kinetics of the glucose-regulated gene expression loop control system of the present invention.
Figure 6 shows the experimental results demonstrating the reversible expression kinetics of the glucose-regulated gene expression loop control system of the present invention.
Figure 7 shows the experimental results showing the impact of different glucose induction times on gene expression within the glucose-regulated gene expression loop control system of the present invention.
Figure 8 shows the experimental results of the glucose-regulated gene expression loop control system regulating the expression of the reporter gene luxCDABE in wild-type mice and type 1 diabetic mice.
Figure 9 shows the experimental results of using probiotic drug factories to regulate the expression and secretion of glucagon-like peptide for the treatment of type 1 diabetic mice.
Figure 10 shows the experimental results of long-term blood glucose control in type 1 diabetic mice using probiotic drug factories regulated to express and secrete glucagon-like peptide.
Figure 11 shows the experimental results of glucose tolerance during the treatment of type 1 diabetes using glucose-regulated probiotic drug factories.
Figure 12 shows the experimental results of the glucose-regulated gene expression loop control system regulating the expression of the reporter gene luxCDABE at the skin wound site of diabetic mice.
Figure 13 shows the experimental results of glucose-regulated induction of human acidic fibroblast growth factor rhaFGF135 expression and secretion by the probiotic drug factory of the present invention.
Figure 14 shows the experimental results of glucose-regulated induction of chemokine CXCL12 expression and secretion by the probiotic drug factory of the present invention.
Figure 15 shows the experimental results of activating the probiotic drug factory at the wound site of diabetic mice, leading to the expression and secretion of human acidic fibroblast growth factor rhaFGF135 and chemokine CXCL12.
Figure 16 shows the experimental results showing the promotion of skin wound healing in diabetic mice by the glucose-regulated probiotic drug factory of the present invention.
Figure 17 shows the blood parameters of mice after the application of the probiotic drug factory at the wound site.
Figure 18 shows the residual of recombinant probiotics in various organs and blood of mice after the in-situ application of the probiotic drug factory at the wound site of the present invention.

### DETAILED DESCRIPTION

The following specific examples and accompanying drawings are given to further elucidate the present invention. These examples are intended solely to illustrate the present invention and do not constitute any limitation on the scope of the present invention. The procedures, conditions, and experimental methods, etc. employed in the implementation of the present invention, unless otherwise explicitly mentioned below, are all general knowledge and common knowledge in the art. There are no specific limitations set by the present invention. The reagents, instruments, etc., as well as experimental methods without specific conditions specified used in the following examples are conducted under routine conditions or recommended conditions by the suppliers.

### Molecular Cloning

Molecular cloning techniques were utilized to construct all expression plasmids in the present invention. The steps are common knowledge in the art.

All primers used for PCR were synthesized by GenScript Biotech Corporation. The expression plasmids constructed in the examples of the present invention were all sequenced, which were performed by GenScript Biotech Corporation. The Phanta Max Super-Fidelity DNA Polymerase used in the examples of the present invention was purchased from Vazyme Biotech Co., Ltd. Endonucleases and T4 DNA ligase were purchased from TaKaRa. The homologous recombinase was obtained from Sangon Biotech (Shanghai) Co., Ltd. Phanta Max Super-Fidelity DNA Polymerase was supplied with the corresponding polymerase buffer and dNTPs upon purchase. Endonucleases, T4 DNA ligase, and homologous recombinase were provided with the respective buffers upon purchase. Yeast extract, tryptone, agar powder, M9 medium, glucose, ampicillin (Amp), and kanamycin (Kan) were purchased from Sangon Biotech (Shanghai) Co., Ltd. DNA Marker DL5000 and DNA Marker DL2000 were obtained from TaKaRa; ethidium bromide (EB) was sourced from Guangdong Genomeditech Company; the plasmid mini-prep kit was obtained from TIANGEN Biotech (Beijing) Co., Ltd. The DNA gel recovery kit and PCR product purification kit were purchased from Kangwei Century Biotech Co., Ltd. The remaining reagents mentioned in the examples, such as anhydrous ethanol and NaCl, were domestic analytical grade products. The steps for gel recovery and purification recovery of DNA fragments were carried out according to the operation manuals of the DNA gel recovery kit and PCR product purification kit (Kangwei Century Biotech Co., Ltd.). The plasmid extraction steps were conducted according to the plasmid mini-prep kit instruction manual (TIANGEN Biotech (Beijing) Co., Ltd.).

### Bacterial Culture and Transformation

In the examples of the present invention, the following bacterial chassis cells and electroporation are used as examples to illustrate the operation of the glucose-regulated gene expression loop control system in prokaryotic cells and in vivo, but it does not limit the scope of protection of the present invention.

Bacterial Culture: EcN was cultured in LB medium supplemented with 100 µg/ml ampicillin and 50 µg/ml kanamycin solution. The bacteria were cultured in a shaker at 37°C and 210 rpm.

Preparation of EcN Competent Cells: All solutions and consumables used for the preparation of competent cells were sterilized by autoclaving. EcN was streaked on antibiotic-free plates and incubated inverted at 37°C for 12-16 hours. A single colony was picked and inoculated into 2 ml of antibiotic-free LB medium in a shake tube and incubated overnight at 37°C and 210 rpm with shaking. Then, 1 ml of the overnight culture was transferred into 100 ml of fresh LB medium and incubated at 37°C and 210 rpm on a shaker until the OD600 reached 0.4-0.6. The culture was then transferred to a centrifuge tube, placed on ice for 15 minutes, and centrifuged at 3500 rpm for 10 minutes at 4°C. The supernatant was discarded, and the pellet was resuspended sequentially in 50 ml and 25 ml of pre-cooled ddH2O, followed by centrifugation at 3500 rpm for 10 minutes at 4°C. The pellet was then resuspended in 25 ml of pre-cooled 10% glycerol, centrifuged at 3500 rpm for 10 minutes at 4°C, and the supernatant was discarded. The final pellet was resuspended in pre-cooled 10% glycerol, aliquoted into 100 µl portions, and stored at -80°C.

Transformation: Transformation of EcN was performed using an optimized electroporation method. Briefly, a mixture of 300-500 ng of pre-cooled plasmid DNA and 100 µl of competent cells was placed at the bottom of an electroporation cuvette. The electroporation parameters were set to 2 mm, 2500 V, and a single pulse was applied. After electroporation, 900 µl of medium was added to the electroporation cuvette, mixed, and then transferred to a sterile EP tube. The cells were incubated at 37°C and 210 rpm for 1 hour, then plated on selective plates containing 100 µg/ml Amp and 50 µg/ml Kan, and cultured at 37°C for 16-20 hours.

### Detection of the Reporter Gene Superfolder Green Fluorescent Protein (sfGFP)

Superfolder GFP (sfGFP) exhibits visible fluorescence under blue light, and its fluorescence intensity can be measured using a Synergy H1 microplate reader. A 100 µl bacterial suspension was added to a black 96-well microplate and placed in the microplate reader. Under the action of excitation light with a wavelength of 480nm, the emission light reading of the bacterial suspension at a wavelength of 520 nm was measured to determine the green fluorescence intensity. Concurrently, a 100 µl of bacterial suspension was added to a clear 96-well microplate, and the absorbance of the bacterial culture at 600 nm was measured using the microplate reader to determine the bacterial cell density. The expression efficiency of the reporter gene was characterized by the ratio of fluorescence intensity to bacterial cell density.

### Extraction of Secreted Proteins

To 2 ml of bacterial culture supernatant, 8 ml of methanol, 2 ml of chloroform, and 8 ml of water were added sequentially, mixed, and centrifuged at 12000 g for 10 minutes. The upper layer was removed, 8 ml of methanol was added, mixed, and centrifuged at 12000 g for 15 minutes. The supernatant was discarded, and the pellet was air-dried and resuspended in 30 µl of PBS buffer.

### Protein Immunoblotting (Western Blotting)

Protein samples were added with 10 µl of 4 × protein loading buffer and subjected to SDS-PAGE electrophoresis. Proteins were transferred to a membrane using the wet electroblotting (250 mA, 1-1.5 hours). The membrane was blocked with TBST + 5% non-fat milk for 1.5 hours, washed three times with TBST, added with the primary antibody, and incubated at 4°C overnight. After washing three times with TBST, the membrane was added with the secondary antibody and incubated at room temperature for 2 hours. Detection was performed using a gel imaging system.

### Example 1: Construction of the Glucose-Regulated Gene Expression Loop Control System

This example includes the construction method for plasmid vectors involved in the glucose-regulated gene expression loop control system. Detailed design schemes and steps are provided in Table 1.

**Table 1: Plasmid Construction Table [00104]**

| **Plasmids** | **Description** |
|---|---|
| pGN11 | The expression vector (P_{hexR1 (PTac-HexO-RBS1)}-sfGFP) for expression of sfGFP driven by the P_{Tac} Promoter: The vector fragment OGN11 (HexO) was obtained by PCR amplification using primers OGN11-1 (SEQ ID NO.16) and OGN11-2 (SEQ ID NO.17) with pYC190 as the template and then seamlessly cloned and connected. |
| pGN69 | The expression vector (P_{BBA23100}-HexR) for expression of HexR driven by the P_{BBA23100} Promoter: The sequence fragment OGN69 (HexR) was obtained by PCR amplification using primers OGN69-1 (SEQ ID NO.18) and OGN69-2 (SEQ ID NO.19) with pHexR as the template, then connected to the pGN53(*Bgl*II/*Kpn*I) vector by seamlessly cloning. |
| pGN89 | The expression vector (P_{Tac}-RBS1-HexR) for expression of HexR driven by the P_{Tac} Promoter. |
| | The sequence fragment OGN69 (HexR) was obtained by PCR amplification using primers OGN69-1 (SEQ ID NO.18) and OGN69-2 (SEQ ID NO.19) with pHexR as the template, and then connected to the pGN15 (*Bgl*II/*Kpn*I) vector by seamlessly cloning. |
| pGN90 | The expression vector (P_{Lac}-HexR) for expression of HexR driven by the P_{Lac} Promoter. |
| | The sequence fragment OGN69 (HexR) was obtained by PCR amplification using primers OGN69-1 (SEQ ID NO.18) and OGN69-2 (SEQ ID NO.19) with pHexR as the template, and then connected to the pGN62 (*Bgl*II/*Kpn*I) vector by seamlessly cloning. |
| pGN227 | The expression vector (P_{hcxR2 (PTac-2-HcxO-RBS1)}-sfGFP) for expression of sfGFP driven by the P_{Tac} Promoter: The v ector fragment OGN227 (2×HexO) was obtained by PCR amplification using primers OGN11-1 (SEQ ID NO.16) and OGN227-2 (SEQ ID NO.20) with pYC190 as the template and then seamlessly cloned and connected. |
| pGN12 | The expression vector (P_{hexR3 (PTac-3×HexO-RBS1)}-sfGFP) for expression of sfGFP driven by the P_{Tac} Promoter: |
| | The vector fragment OGN12 (3×HexO) was obtained by PCR amplification using primers OGN12-1 (SEQ ID NO.21) and OGN227-2 (SEQ ID NO.20) with pYC190 as the template and then seamlessly cloned and connected. |
| pGN228 | The expression vector (P_{hexR4 (PTac-4×HexO-RBS1)}-sfGFP) for expression of sfGFP driven by the P_{Tac} Promoter: |
| | The vector fragment OGN228 (4×HexO) was obtained by PCR amplification using primers OGN11-1 (SEQ ID NO.16) and OGN228-2 (SEQ ID NO.22) with pYC190 as the template and then seamlessly cloned and connected. |
| pGN229 | The expression vector (P_{hexR5 (PTac-5×HexO-RBS1)}-sfGFP) for expression of sfGFP driven by the P_{Tac} Promoter: The vector fragment OGN229 (5×HexO) was obtained by PCR amplification using primers OGN229-1 (SEQ ID NO.23) and OGN228-2 (SEQ ID NO.22) with pYC190 as the template and then seamlessly cloned and connected. |
| pGN220 | The expression vector (P_{hexR6 (PLac-HexO-RBS1)}-sfGFP) for expression of sfGFP Driven by the P_{Lac} Promoter: |
| | The vector fragment OGN220 was obtained by PCR amplification using primers OGN220-1 (SEQ ID NO.24) and OGN220-2 (SEQ ID NO.25) with pGN11 as the template and then seamlessly cloned and connected. |
| pGN221 | The expression vector (P_{hexR7 (PLac-2×HexO-RBS1)}-sfGFP) for expression of sfGFP driven by the P_{Lac} Promoter: |
| | The vector fragment OGN220 was obtained by PCR amplification using primers OGN220-1 (SEQ ID NO.24) and OGN220-2 (SEQ ID NO.25) with pGN227 as the template and then seamlessly cloned and connected. |
| pGN222 | The expression vector (P_{hcxR8 (PLac-3×HcxO-RBS1)}-sfGFP) for expression of sfGFP driven by the P_{Lac} Promoter: |
| | The vector fragment OGN220 was obtained by PCR amplification using primers OGN220-1 (SEQ ID NO.24) and OGN220-2 (SEQ ID NO.25) with pGN12 as the template and then seamlessly cloned and connected. |
| pGN223 | The expression vector (P_{hexR9 (PLac-4×HexO-RBS1)}-sfGFP) for expression of sfGFP driven by the P_{Lac} Promoter: |
| | The vector fragment OGN220 was obtained by PCR amplification using primers OGN220-1 (SEQ ID NO.24) and OGN220-2 (SEQ ID NO.25) with pGN228 as the template and then seamlessly cloned and connected. |
| pGN224 | The expression vector (P_{hexR10 (PLac-5×HexO-RBS1)}-sfGFP) for expression of sfGFP driven by the P_{Lac} Promoter: |
| | The vector fragment OGN220 was obtained by PCR amplification using primers OGN220-1 (SEQ ID NO.24) and OGN220-2 (SEQ ID NO.25) with pGN229 as the template and then seamlessly cloned and connected. |
| pGN231 | The expression vector (P_{Tac}-RBS2-HexR) for expression of HexR driven by the P_{Tac} Promoter: |
| | The vector fragment OGN231 was obtained by PCR amplification using primers OGN231-1 (SEQ ID NO.26) and OGN232-1 (SEQ ID NO.27) with pGN89 as the template and then seamlessly cloned and connected. |
| pGN232 | The expression vector (P_{Tac}-RBS3-HexR) for expression of HexR driven by the P_{Tac} Promoter: |
| | The vector fragment OGN232 was obtained by PCR amplification using primers OGN232-1 (SEQ ID NO.28) and OGN232-2 (SEQ ID NO.29) with pGN89 as the template and then seamlessly cloned and connected. |
| pGN233 | The expression Vector (P_{Tac}-RBS4-HexR) for expression of HexR driven by the P_{Tac} Promoter: The vector |
| | fragment OGN233 was obtained by PCR amplification using primers OGN233-1 (SEQ ID NO.30) and OGN233-2 (SEQ ID NO.31) with pGN89 as the template and then seamlessly cloned and connected. |
| pGN65 | The expression vector (P_{hexR4 (PTac-4×HexO-RBS3)}-LuxCDABE) for expression of LuxCDABE driven by the P_{Tac} Promoter. |
| | The sequence fragment OGN65 (LuxCDABE) was obtained by PCR amplification using primers OGN65-1 (SEQ ID NO.32) and OGN65-2 (SEQ ID NO.33) with pBEN276 as the template, and then connected to the pGN228 (*Nco*I*lEcoR*I) vector by seamlessly cloning. |
| pGN237 | The expression vector (P_{hexR4 (PTac-4×HexO-RBS3)}-phoA-GLP1) for expression of phoA-GLP-1 driven by the P_{Tac} Promoter. |
| | The sequence fragment OGN237 (phoA-GLP-1) was obtained by PCR amplification using primers OGN237-1 (SEQ ID NO.34), OGN237-2 (SEQ ID NO.35), and OGN237-3 (SEQ ID NO.36) with pGLP1 as the template, and then connected to the pGN228 (*Neo*I/*Not*I) vector by seamlessly cloning. |
| pGN238 | The expression vector (P_{hexR4 (PTac-4×HexO-RBS3)}-ompA-GLP1) for expression of ompA-GLP-1 driven by the P_{Tac} Promoter. |
| | The sequence fragment OGN238 (ompA-GLP-1) was obtained by PCR amplification using primers OGN238-1 (SEQ ID NO.37), OGN238-2 (SEQ ID NO.38), and OGN237-3 (SEQ ID NO.36) with pGLP1 as the template, and then connected to the pGN228 (*Nco*I/*Not*I) vector by seamlessly cloning. |
| pGN239 | The expression vector (P_{hexR4 (PTac-4×HexO-RBS3)}-ompT-GLP1) for expression of ompT-GLP-1 driven by the P_{Tac} Promoter. |
| | The sequence fragment OGN239 (ompT-GLP-1) was obtained by PCR amplification using primers OGN239-1 (SEQ ID NO.39), OGN239-2 (SEQ ID NO.40), and OGN237-3 (SEQ ID NO.36) with pGLP1 as the template, and then connected to the pGN228 (NcoI/NotI) vector by seamlessly cloning. |
| pGN241 | The expression vector (P_{hexR4 (PTac-4×HexO-RBS3)}-lamB-GLP1) for expression of lamB-GLP-1 driven by the P_{Tac} Promoter. |
| | The sequence fragment OGN241 (lamB-GLP-1) was obtained by PCR amplification using primers OGN241-1 (SEQ ID NO.41), OGN241-2 (SEQ ID NO.42), and OGN237-3 (SEQ ID NO.36) with pGLP1 as the template, and then connected to the pGN228 (*Nco*I/*Not*I) vector by seamlessly cloning. |
| pGN242 | The expression vector (P_{hexR4 (PTac-4×HexO-RBS3)}-pelB-GLP1) for expression of pelB-GLP-1 driven by the P_{Tac} Promoter. |
| | The sequence fragment OGN242 (pelB-GLP-1) was obtained by PCR amplification using primers OGN242-1 (SEQ ID NO.43), OGN242-2 (SEQ ID NO.44), and OGN237-3 (SEQ ID NO.36) with pGLP1 as the template, and then connected to the pGN228 (*Nco*I/*Not*I) vector by seamlessly cloning. |
| pGN243 | The expression vector (P_{hexR4 (PTac-4×HexO-RBS3)}-yopE-GLP1) for expression of yopE-GLP-1 driven by the P_{Tac} Promoter. |
| | The sequence fragment OGN243 (yopE-GLP-1) was obtained by PCR amplification using primers OGN243-1 (SEQ ID NO.45), OGN243-2 (SEQ ID NO.46), and OGN237-3 (SEQ ID NO.36) with pGLP1 as the template, and then connected to the pGN228 (*Nco*I/*Not*I) vector by seamlessly cloning. |
| pGN288 | The expression vector (P_{hexR4 (PTac-4×HexO-RBS3)}-yopE-EK-GLP1) for expression of EK-GLP-1 driven by the P_{Tac} Promoter. |
| | The vector fragment OGN288 (EK-GLP-1) was obtained by PCR amplification using primers OGN288-1 (SEQ ID NO.47) and OGN288-2 (SEQ ID NO.48) with pGN243 as the template and then seamlessly cloned and connected. |
| pGN308 | The expression vector (P_{hexR4 (PTac-4×HexO-RBS3)}-yopE-EK-GLP1-Fc) for expression of GLP-1-Fc driven by the P_{Tac} Promoter. |
| | The sequence fragment OGN308 (GLP-1-Fc) was obtained by PCR amplification using primers OGN308-1 (SEQ ID NO.49) and OGN308-2 (SEQ ID NO.50) with pGLP1-Fc as the template. The vector fragment OGN308 was obtained by PCR amplification using primers OGN308-3 (SEQ ID NO.51) and OGN308-4 (SEQ ID NO.52) with pGN288 as the template. The two fragments were connected by seamlessly cloning. |
| pGN299 | The expression vector (P_{hexR4 (PTac-4×HexO-RBS3)}-yopE-rhaFGF135) for expression of yopE-rhaFGF135 driven by the P_{Tac} Promoter. The sequence fragment OGN299 (yopE-rhaFGF135) was obtained by PCR amplification using primers OGN299-1 (SEQ ID NO.53), OGN299-2 (SEQ ID NO.54), and OGN299-3 (SEQ ID NO.55) with prhaFGF135 as the template, and then connected to the pGN228 (*Neo*I/*Not*I) vector by seamlessly cloning. |
| pXG18 | The expression vector (P_{hexR4 (PTac-4x HexO-RBS3)}-OmpA-CXCL12) for expression of ompA-CXCL12 driven by the P_{Tac} Promoter. The sequence fragment OXG18 (yopE-rhaFGF135) was obtained by PCR amplification using primers OXG18-1 (SEQ ID NO.56), OXG18-2 (SEQ ID NO.57), and OXG18-3 (SEQ ID NO.58) with pCXCL12 as the template, and then connected to the pGN228 (*Neo*I/*Not*I) vector by seamlessly cloning. |
| pGN300 | The expression vector (P_{hexR4 (PTac-4xHexO-RBS3)}-yopE-rhaFGF135-RBS-OmpA-CXCL12) for expression of yopE-rhaFGF135 and ompA-CXCL12 driven by the P_{Tac} Promoter. The sequence fragment OGN300 (RBS-OmpA-CXCL12) was obtained by PCR amplification using primers OGN300-1 (SEQ ID NO.59) and OGN300-2 (SEQ ID NO.60) with pXG18 as the template, and then connected to the pGN299 (*EcoR*I/*Xho*I) vector by seamlessly cloning. |
| pGN306 | The expression vector (P_{hexR9 (PLac-4×HexO-RBS1)}-mPDX1) for expression of mPDX1 driven by the P_{Lac} Promoter. |
| | The sequence fragment OGN306 (mPDX1) was obtained by PCR amplification using primers OGN306-1 (SEQ ID NO.62) and OGN306-2 (SEQ ID NO.63) with pmPDX1 as the template. The vector fragment OGN306 was obtained by PCR amplification using primers OGN306-3 (SEQ ID NO.64) and OGN306-4 (SEQ ID NO.65) with pGN223 as the template. The two fragments were connected by seamlessly cloning. |
| pGN307 | The expression vector (P_{hexR9 (PLac-4×Hexo-RBS1)}-hPDX1) for expression of hPDX1 driven by the P_{Lac} Promoter. |
| | The sequence fragment OGN307 (hPDX1) was obtained by PCR amplification using primers OGN307-1 (SEQ ID NO.67) and OGN307-2 (SEQ ID NO.68) with phPDX1 as the template. The vector fragment OGN307 was obtained by PCR amplification using primers OGN307-3 (SEQ ID NO.69) and OGN307-4 (SEQ ID NO.70) with pGN223 as the template. The two fragments were connected by seamlessly cloning. |
| pXG58 | The expression vector (P_{hcxR9 (PLac-4×HcxO-RBS1)}-GLP-1-Fc-hPDX1) for expression of yopE-EK-GLP-1-Fc and yopE-hPDX1 driven by the P_{Lac} Promoter. The sequence fragment OXG58 (hPDX-1) was obtained by PCR amplification using primers OXG58-1 (SEQ ID NO.71) and OXG58-2 (SEQ ID NO.72) with phPDX-1 as the template. The vector fragment OXG58 was obtained by PCR using primers OXG58-3 (SEQ ID NO.73) and OXG58-4 (SEQ ID NO.74) with pGN308 as the template. The two fragments were connected by seamlessly cloning. |
| pGN314 | The expression vector (P_{hexR4 (PTac-4xHexO-RBS3)}-yopE-rhaFGF135-RBS-OmpA-CXCL12-RBS-yopE-hIL4) for expression of yopE-rhaFGF135, ompA-CXCL12 and yopE-hIL4 driven by the P_{Tac} Promoter. The sequence fragment OGN314 (RBS-yopE-hIL4) was obtained by PCR amplification using primers OGN314-1 (SEQ ID NO.76) and OGN314-2 (SEQ ID NO.77) with RBS-yopE-hIL4 as the template, and then connected to the pGN300 (Bgl II/*Kpn I*) vector by seamlessly cloning. |

Primer Pairs (Numbers 1-30), as shown in Table 2.

| **Number** | Primer Pairs |
|---|---|
| 1 | Primer OGN11-1 (SEQ ID NO.16) and OGN11-2 (SEQ ID NO.17) |
| 2 | Primer OGN69-1 (SEQ ID NO.18) and OGN69-2 (SEQ ID NO.19) |
| 3 | Primer OGN11-1 (SEQ ID NO.16) and OGN227-2 (SEQ ID NO.20) |
| 4 | Primer OGN12-1 (SEQ ID NO.21) and OGN227-2 (SEQ ID NO.20) |
| 5 | Primer OGN11-1 (SEQ ID NO.16) and OGN228-2 (SEQ ID NO.22) |
| 6 | Primer OGN229-1 (SEQ ID NO.23) and OGN228-2 (SEQ ID NO.22) |
| 7 | Primer OGN220-1 (SEQ ID NO.24) and OGN220-2 (SEQ ID NO.25) |
| 8 | Primer OGN231-1 (SEQ ID NO.26) and OGN232-1(SEQ ID NO.27) |
| 9 | Primer OGN232-1 (SEQ ID NO.28) and OGN232-2 (SEQ ID NO.29) |
| 10 | Primer OGN233-1 (SEQ ID NO.30) and OGN233-2 (SEQ ID NO.31) |
| 11 | Primer OGN65-1 (SEQ ID NO.32) and OGN65-2 (SEQ ID NO.33) |
| 12 | Primer OGN237-1 (SEQ ID NO.34), OGN237-2 (SEQ ID NO.35) and OGN237-3 (SEQ ID NO.36) |
| 13 | Primer OGN238-1 (SEQ ID NO.37), OGN238-2 (SEQ ID NO.38) and OGN237-3 (SEQ ID NO.36) |
| 14 | Primer OGN239-1 (SEQ ID NO.39), OGN239-2 (SEQ ID NO.40) and OGN237-3 (SEQ ID NO.36) |
| 15 | Primer OGN241-1 (SEQ ID NO.41), OGN241-2 (SEQ ID NO.42) and OGN237-3 (SEQ ID NO.36) |
| 16 | Primer OGN242-1 (SEQ ID NO.43), OGN242-2 (SEQ ID NO.44) and OGN237-3 (SEQ ID NO.36) |
| 17 | Primer OGN243-1 (SEQ ID NO.45), OGN243-2 (SEQ ID NO.46) and OGN237-3 (SEQ ID NO.36) |
| 18 | Primer OGN288-1 (SEQ ID NO.47) and OGN288-2 (SEQ ID NO.48) |
| 19 | Primer OGN308-1 (SEQ ID NO.49) and OGN308-2 (SEQ ID NO.50) |
| 20 | Primer OGN308-3 (SEQ ID NO.51) and OGN308-4 (SEQ ID NO.52) |
| 21 | Primer OGN299-1 (SEQ ID NO.53), OGN299-2 (SEQ ID NO.54) and OGN399-3 (SEQ ID NO.55) |
| 22 | Primer OXG18-1 (SEQ ID NO.56), OXG18-2 (SEQ ID NO.57) and OXG18-3 (SEQ ID NO.58) |
| 23 | Primer OGN300-1 (SEQ ID NO.59) and OGN300-2 (SEQ ID NO.60) |
| 24 | Primer OGN306-1 (SEQ ID NO.62) and OGN306-2 (SEQ ID NO.63) |
| 25 | Primer OGN306-3 (SEQ ID NO.64) and OGN306-4 (SEQ ID NO.65) |
| 26 | Primer OGN307-1 (SEQ ID NO.67) and OGN307-2 (SEQ ID NO.68) |
| 27 | Primer OGN307-3 (SEQ ID NO.69) and OGN307-4 (SEQ ID NO.70) |
| 28 | Primer OXG58-1 (SEQ ID NO.71) and OXG58-2 (SEQ ID NO.72) |
| 29 | Primer OXG58-3 (SEQ ID NO.73) and OXG58-4 (SEQ ID NO.74) |
| 30 | Primer OGN314-1 (SEQ ID NO.76) and OGN314-2 (SEQ ID NO.77) |

**Example 2:** Optimization Study of the Glucose-Regulated Gene Expression Loop Control System in EcN. In this example, the optimization study of the glucose-regulated gene expression loop control system in EcN is performed by combining the expression vectors for expression of HexR driven by different promoters with the glucose-induced strong promoter PHexR1.

### Step 1: Plasmid Construction

The detailed method for plasmid construction in this example is provided in Table 1.

### Step 2: Transformation

The transformation system in this example is divided into four groups: including pGN11, pGN11 and pGN69, pGN11 and pGN89, pGN11 and pGN90. Each group of plasmids was electroporated into EcN electrocompetent cells.

### Step 3: Culture

Positive single colonies were picked and transferred to liquid LB medium, then cultured at 37°C and 210 rpm for 12 hours.

### Step 4: Detection of Reported Gene sfGFP Expression Levels

100 µL of bacterial suspension was added to black 96-well microplates and clear 96-well microplates. The fluorescence intensity and bacterial cell density were measured using a microplate reader. The expression efficiency of the reporter gene was represented by the ratio of fluorescence intensity to bacterial cell density.

### Experimental Results

As shown in Figure 2, the expression of the reporter gene sfgfp was inhibited after the exogenous expression of HexR repressor protein, with the strongest repression of the expression of the reporter gene observed when HexR (pGN89) was driven by the Tac promoter.

**Example 3:** Optimization Study of the Glucose-Regulated Gene Expression Loop Control System in EcN. In this example, the optimization study of the glucose-regulated gene expression loop control system in EcN is performed by combining the expression vector for expression of HexR driven by the PTac promoter with five different response elements.

### Step 1: Plasmid Construction

The detailed method for plasmid construction in this example is provided in Table 1.

### Step 2: Transformation

The transformation system in this example is divided into five groups: including pGN89 and pGN11, pGN89 and pGN227, pGN89 and pGN12, pGN89 and pGN228, pGN89 and pGN229. Each group of plasmids was electroporated into EcN electrocompetent cells.

### Step 3: Culture

The specific steps are the same as in Example 2 of the present invention.

### Step 4: Induction

The culture was centrifuged at 5000 rpm for 5 minutes. The bacterial pellet was resuspended in an equal volume of M9 medium and pipetted evenly, then distributed into each well of a 48-well plate at the culture volume of 500 µL. The appropriate concentration of sterile glucose solution was added to different experimental groups, cultured with shaking at 37°C and 150 rpm for 12 hours.

### Step 5: Detection of Reported Gene sfGFP Expression Levels

The specific steps are the same as in Example 2 of the present invention.

### Experimental Results

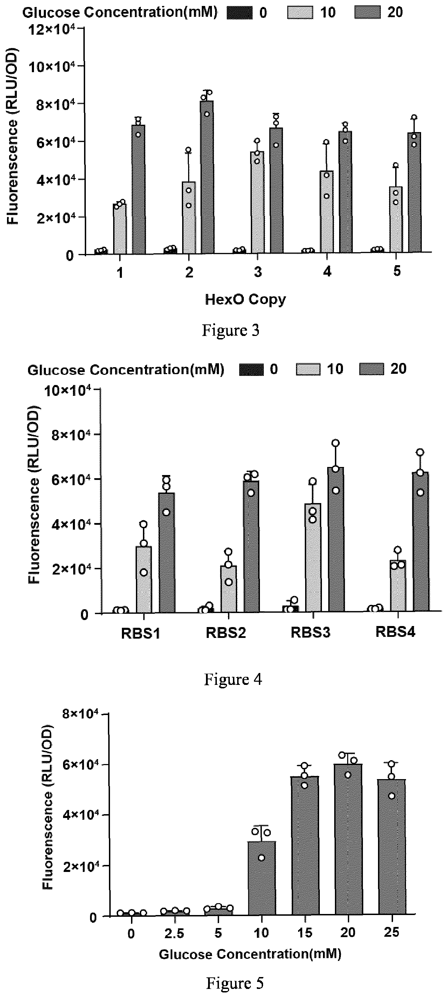

As shown in Figure 3, different optimization combinations of the glucose-regulated gene expression loop control system can activate the expression of the reporter gene sfGFP, but the induction effects vary. Among the combinations, the combination of pGN89 and pGN228 achieved the best induction multiple.

**Example 4:** Optimization Study of the Glucose-Regulated Gene Expression Loop Control System in EcN. In this example, the optimization study of the glucose-regulated gene expression loop control system in EcN is performed by combining different RBS-regulated HexR expression vectors with the glucose-induced strong promoter PHexR4.

### Step 1: Plasmid Construction

The detailed method for plasmid construction in this example is provided in Table 1.

### Step 2: Transformation

The transformation system in this example is divided into four groups: including pGN89 and pGN228, pGN231 and pGN228, pGN232 and pGN228, pGN233 and pGN228. Each group of plasmids was electroporated into EcN electrocompetent cells.

### Step 3: Culture

The specific steps are the same as in Example 2 of the present invention.

### Step 4: Induction

The specific steps are the same as in Example 3 of the present invention.

### Step 5: Detection of Reported Gene sfGFP Expression Levels

The specific steps are the same as in Example 2 of the present invention.

### Experimental Results

As shown in Figure 4, different optimization combinations of the glucose-regulated gene expression loop control system can activate the expression of the reporter gene sfGFP, but the induction effects of the system vary. Among the combinations, the combination of pGN233 and pGN228 achieved the best induction multiple.

### Example 5: Study on the Fine-Tuning Expression Kinetics Characteristics of the Glucose-Regulated Gene Expression Loop Control System

Step 1: The engineered bacteria obtained from Example 4 with the best induction multiple were inoculated and cultured, centrifuged at 5000 rpm for 5 minutes. The bacterial pellet was resuspended in an equal volume of M9 medium and pipetted evenly, then distributed into each well of a 48-well plate at the culture volume of 500 µL. Different final concentrations of sterile glucose solution was added to different experimental groups for induction, and cultured with shaking at 37°C and 150 rpm for 12 hours.

Step 2: Detection of Reporter Gene sfGFP Expression Levels (follow the same steps as in Example 2 of the present invention).

Experimental Results: As shown in Figure 5, the engineered bacteria of the glucose-regulated gene expression loop control system exhibit precise regulation by glucose, indicating dose-dependent induction of gene expression, which further demonstrates the fine-tuning expression kinetics characteristics of the system.

### Example 6: Study on the Reversible Expression Kinetics Characteristics of the Glucose-Regulated Gene Expression Loop Control System

Step 1: The engineered bacteria obtained from Example 4 with the best induction multiple were inoculated and cultured, centrifuged at 5000 rpm for 5 minutes. The bacterial pellet was resuspended in an equal volume of M9 medium and pipetted evenly, then distributed into each well of a 48-well plate at the culture volume of 500 µL.

Step 2: The "ON-OFF-ON" experimental group was induced by adding sterile glucose solution with a final concentration of 20 mM at 0 h; at the 2nd hour, the original medium was removed by centrifugation, glucose-free M9 medium was added for resuspending and culturing; at the 7th hour, the medium was removed by centrifugation, M9 medium containing a final concentration of 20 mM glucose was added for resuspending and culturing. The "OFF-ON-OFF" experimental group was induced by adding no glucose at 0 h; at the 2nd hour, a sterile glucose solution with a final concentration of 20 mM was added for induction; at the 7th hour, the original medium was removed by centrifugation, glucose-free M9 medium was added for resuspending and culturing. For both experimental groups, the bacterial suspension was aspirated every 1 hour to detect the expression levels of the reporter gene sfGFP (follow the same steps as in Example 2 of the present invention).

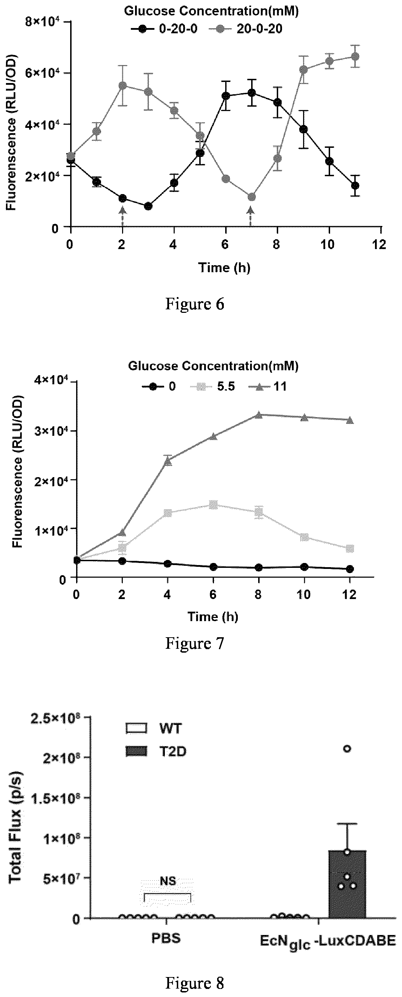

Experimental Results: As shown in Figure 6, the expression of the system's genes can be turned on or off by controlling the presence or absence of glucose, demonstrating that the glucose-regulated gene expression loop control system has good reversibility.

### Example 7: Study on the Control of Gene Expression by Different Induction Times of Glucose in the Glucose-Regulated Gene Expression Loop Control System

Step 1: The engineered bacteria obtained from Example 4 with the best induction multiple was inoculated and cultured, centrifuged at 5000 rpm for 5 minutes. The bacterial pellet was resuspended in an equal volume of M9 medium and pipetted evenly, then distributed into each well of a 48-well plate at the culture volume of 500 µL. A sterile glucose solution with a final concentration of 20 mM was added to different experimental groups, and incubated with shaking at 37°C and 150 rpm for different times (0 h, 2 h, 4 h, 6 h, 8 h, 10 h, 12 h).

### Step 2: Detect the reporter gene sfGFP Expression Levels (follow the same steps as in Example 2 of this invention).

Experimental Results: As shown in Figure 7, the gene expression level of the system is regulated by the induction time of glucose, indicating that the glucose-regulated gene expression loop control system has good controllability.

### Example 8: Establishment of a Type 1 Diabetes Mouse Model Using Streptozotocin (STZ) Injection

### Step 1: Fasting

Before administration, 40 male C57BL/6J mice weighing approximately 25 grams each were selected and fasted for up to 16 hours.

### Step 2: Administration

STZ was dissolved in citrate buffer (0.1 mol/L, pH 4.5), and then injected intraperitoneally into mice at a dosage of 40-50 mg/kg for 5 consecutive days. Since STZ is easily degraded, it is essential to keep the solution at low temperature and away from light throughout the process, and the injections need to be performed quickly.

### Step 3: Blood Glucose Measurement

On the 9th day, the blood glucose levels of the mice were measured after 4 hours of fasting. Mice with blood glucose levels higher than 16.7 mM were considered to have successfully established the model.

### Example 9: Study on the Regulation of the Reporter Gene luxCDABE Expression in Type 1 Diabetic Mice Using the Glucose-Regulated Gene Expression Loop Control System

### Step 1: Plasmid Construction

The detailed method for plasmid construction in this example is provided in Table 1.

### Step 2: Transformation

pGN65 and pGN233 were electroporated into EcN electrocompetent cells.

### Step 3: Culture

The specific steps are the same as in Example 2 of the present invention.

### Step 4: Cell Collection

The culture was centrifuged at 5000 rpm for 5 minutes, then the cells were wash ed three times with sterile PBS and resuspended to a concentration of 10⁹ CFU/100 µl

### Step 5: Oral Administration

The bacterial suspension was administrated to both wild-type mice and type 1 diabetic mice (after 4 hours of fasting), 100 µl (10⁹ CFU engineered bacteria/100 µl) per mouse.

### Step 6: Detection of luxCDABE Expression levels in Mice

Six hours after oral administration, the bioluminescence signals were detected using an in vivo imaging system for small animals.

### Experimental Results

As shown in Figure 8, the glucose-regulated gene expression loop control system can activate gene expression in type 1 diabetic mice but not in wild-type mice.

### Example 10: Construction and Measurement of Engineered Bacteria for Glucose-Regulated Expression and Secretion of Glucagon-Like Peptide

### Step 1: Plasmid Construction

The detailed method for plasmid construction in this example is provided in Table 1.

### Step 2: Transformation

The specific steps are the same as in Example 2 of the present invention. The transformation system in this example is divided into eight groups: including pGN237 and pGN233, pGN238 and pGN233, pGN239 and pGN233, pGN241 and pGN233, pGN242 and pGN233, pGN243 and pGN233, pGN288 and pGN233, pGN308 and pGN233. Each group of plasmids was electroporated into EcN electrocompetent cells.

### Step 3: Identification of Glucagon-Like Peptide Expression and Secretion

The amplified engineered bacteria were inoculated and induced with 20 mM glucose. After 12 hours, the intracellular and extracellular levels of glucagon-like peptide were detected. The engineered bacteria with good induction and secretion effects were amplified and preserved.

### Example 11: Study on the Treatment of Type 2 Diabetic (dbdb) Mice Using Probiotics Regulating the Expression and Secretion of Glucagon-Like Peptide

In this example, Type 2 diabetic mice were taken as an example to demonstrate the closed-loop therapeutic function of the probiotic glucose sensor for diabetes, without limiting the scope of the protection of the present invention. The specific steps are as follows:

### Step 1: Cell Collection

The engineered bacteria selected in Example 10 with good glucagon-like peptide secretion effects and the engineered bacteria selected in Example 9 of the present invention for regulating the expression of the reporter gene luxCDABE were amplified and cultured. The culture was centrifuged at 5000 rpm for 5 minutes, the cells were washed three times with sterile PBS, and resuspended to a concentration of 10⁹ CFU/100 µl.

### Step 2: Oral Administration

The PBS and the two engineered bacterial suspensions amplified in Step 1 were administered to Type 2 diabetic mice (after 4 hours of fasting), 100 µl (10⁹ CFU engineered bacteria/100 µl) per mouse.

### Step 3: Blood Glucose Measurement

After 24 hours of oral administration, blood glucose test strips were used to measure the blood glucose levels of the mice (after 4 hours of fasting). Simultaneously the blood glucose levels of wild-type mice were measured as a control.

### Experimental Results

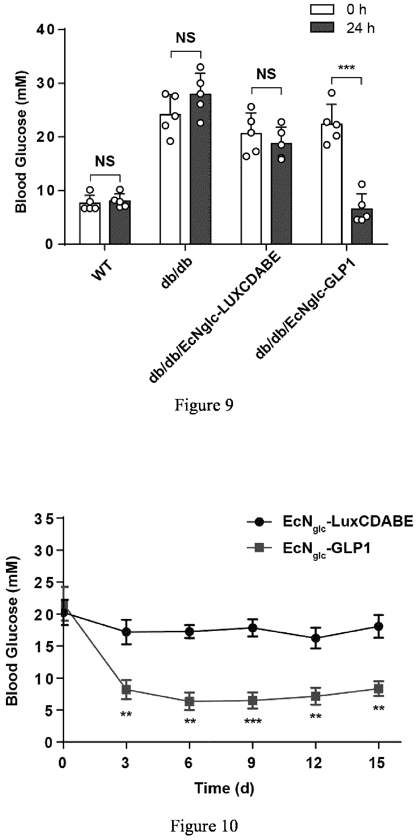

As shown in Figure 9, oral administration of probiotics regulating the expression and secretion of glucagon-like peptide can reduce blood glucose levels in Type 2 diabetic mice.

### Example 12: Long-Term Study on the Effects of Probiotics Regulating the Expression and Secretion of Glucagon-Like Peptide in the treatment of Type 2 Diabetic Mice

### Step 1: Cell Collection

The specific steps are the same as in Example 11 of the present invention.

### Step 2: Oral Administration

The engineered bacterial suspensions were administered as described in Example 11 of the present invention every 24 hours for 15 consecutive days.

### Step 3: Blood Glucose Measurement

The specific steps are the same as in Example 11 of the present invention.

### Experimental Results

As shown in Figure 10, oral administration of probiotics regulating the expression and secretion of glucagon-like peptide can maintain blood glucose homeostasis in Type 2 diabetic mice over a long-term period.

### Example 13: Study on Glucose Tolerance During the Treatment of Type 2 Diabetes with Glucose-Regulated Probiotic Drug Factories

This example was conducted on Type 2 diabetic model mice following the treatment described in Example 12 of the present invention. The specific experimental method for assessing glucose tolerance is as follows:

### Step 1: Fasting

The model mice were subjected to 16 hours of fasting.

### Step 2: Preparation of Glucose Solution

A glucose solution was prepared to a concentration of 125 mg/ml.

### Step 3: Measurement of Blood Glucose Levels

The blood glucose levels of the mice were measured at zero point (0 minute), then the glucose at a dose of 1.25 g/kg was injected intraperitoneally. Subsequently, the blood glucose levels of the mice were measured at 30, 60, 90, and 120 minutes.

### Experimental Results

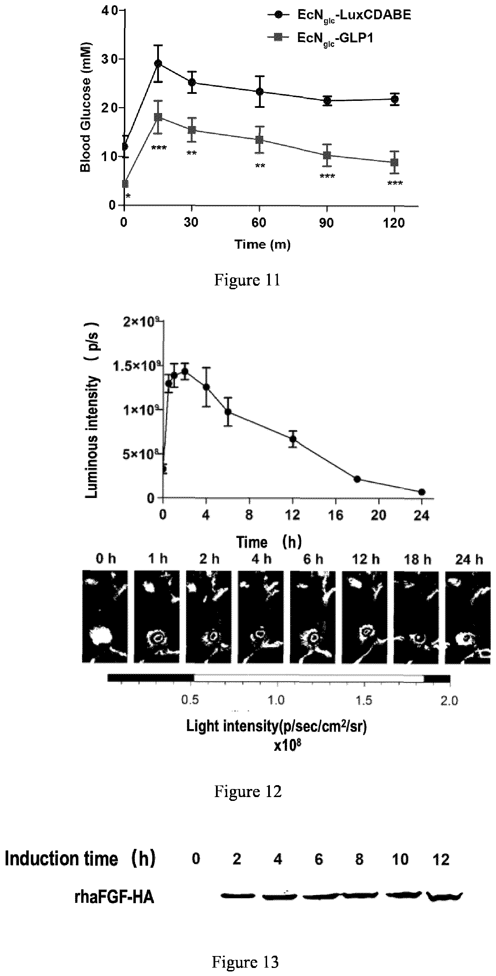

As shown in Figure 11, compared to the control group, the treatment group exhibited significant improvement and control of hyperglycemia. This indicates that probiotics regulating the expression and secretion of glucagon-like peptide have a significant effect on the treatment of Type 2 diabetes.

### Example 14: Skin Wound Modeling in Diabetic Mice

### Step 1: Anesthesia

40 type 1 diabetic model mice were selected and anesthetized using inhalation anesthesia with the gaseous anesthetic isoflurane.

### Step 2: Hair Removal

The hair was removed from the backs of the mice using small animal electric clippers and depilatory cream, then cleaned with alcohol wipes.

### Step 3: Full-Thickness Skin Loss

Two identical circular areas (approximately 30 mm² each) were marked on both sides of the mice back spine where hair was removed. The skin and subcutaneous tissue were cut along the marked areas down to the fascia layer, ensuring that the two cut parts were separated by intact skin. The back wounds of the mice were randomly divided into groups.

### Example 15: Study on the Regulation of Reporter Gene luxCDABE Expression by Glucose-Regulated Probiotic Drug Factories in Skin Wounds of Type 1 Diabetic Mice

### Step 1: Transformation

### pGN65 and pGN233 were electroporated into electrocompetent EcN cells.

### Step 2: Culture

A positive single colony was picked, inoculated into liquid LB medium, and cultured at 37°C and 210 rpm for 12 hours.

### Step 3: Cell Collection

The culture was centrifuged at 5000 rpm for 5 minutes, the cells were washed three times with sterile PBS, and resuspended to a concentration of 10⁹ CFU/100 µl.

### Step 4: In Situ Skin Drip Application

The bacterial suspension was dripped onto the skin wounds of type 1 diabetic mice, 10 µL (10⁹ CFU of engineered bacteria) per mouse.

### Step 5: Detection of luxCDABE Expression Levels in Skin Wounds

After the engineered bacteria were dripped, the bioluminescence signals were detected using an in vivo imaging system for small animals.

### Experimental Results

As shown in Figure 12, the glucose-regulated gene expression loop control system can continuously activate gene expression in the skin wounds of diabetic mice, and the activation can last for approximately 24 hours.

### Example 16: Construction and Measurement of Probiotic Drug Factories for Glucose-Regulated Expression and Secretion of Human Acidic Fibroblast Growth Factor rhaFGF135

### Step 1: Plasmid Construction

The construction of plasmids in this example is detailed in Table 1.

### Step 2: Transformation

pGN299 and pGN233 were electroporated into electrocompetent EcN cells.

### Step 3: Identification of rhaFGF135 Expression and Secretion

The amplified engineered bacteria were inoculated and induced with 20 mM glucose. After 12 hours, the culture was centrifuged to collect the supernatant, the protein in the supernatant was extracted, and the content of rhaFGF135 was detected using western bloting. The engineered bacteria with good induction secretion effects were amplified and preserved.

### Experimental Results

As shown in Figure 13, glucose can regulate the expression and secretion of rhaFGF135.

### Example 17: Construction and Measurement of Probiotic Drug Factories for Glucose-Regulated Expression and Secretion of Chemokine CXCL12

### Step 1: Plasmid Construction

The construction of plasmids in this example is detailed in Table 1.

### Step 2: Transformation

pXG22 and pGN233 were electroporated into electrocompetent EcN cells.

### Step 3: Identification of CXCL12 Expression and Secretion

The amplified engineered bacteria were inoculated and induced with 20 mM glucose. After 12 hours, the culture was centrifuged to collect the supernatant, the protein in the supernatant was extracted, and the content of CXCL12 was detected using western blotting. The engineered bacteria with good induction secretion effects were amplified and preserved.

### Experimental Results

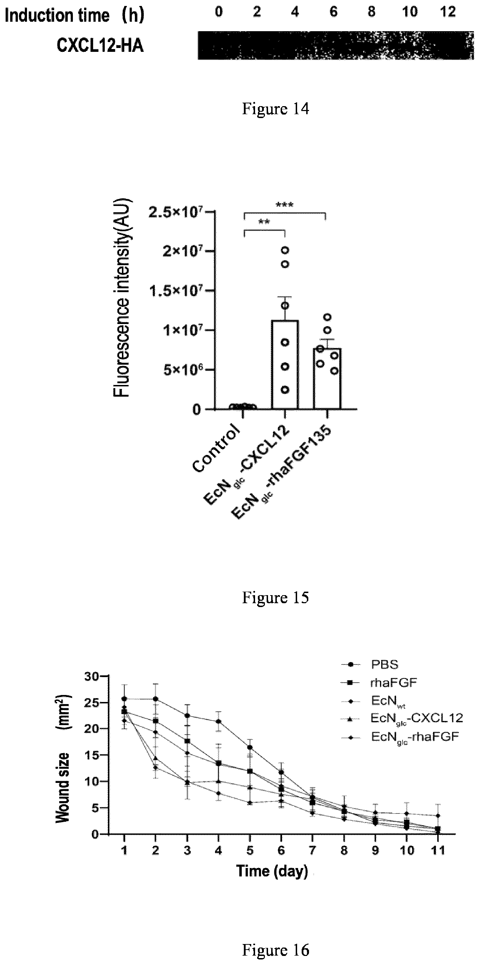

As shown in Figure 14, glucose can regulate the expression and secretion of CXCL12.

### Example 18: Verification of Activation of Probiotic Drug Factories in the Wounds of Diabetic Mice for Expression and Secretion of Human Acidic Fibroblast Growth Factor rhaFGF135 and Chemokine CXCL12

### Step 1: Bacterial Culture

Probiotics for expression and secretion of glucose-regulated human acidic fibroblast growth factor rhaFGF135 and chemokine CXCL12, as well as wild-type EcN, were inoculated into liquid LB medium, cultured at 37°C and 210 rpm for 12 hours.

### Step 2: Cell Collection

The specific steps are the same as in Example 4 of the present invention.

### Step 3: In Situ Skin Drip Application

The specific steps are the same as in Example 4 of the present invention.

### Step 4: Skin Tissue Sampling

After 6 hours, the mice were killed and skin were cut from the wounds of the mice.

### Step 5: Tissue Sectioning

The skin tissues were fixed in 4% paraformaldehyde for 24 hours, then rinsed with running water overnight. The skin was immersed sequentially in 30%, 50%, 75%, 85%, 95%, 100% ethanol, a mixture of 50% ethanol and 50% xylene for 1 hour each, and permeabilized in 100% xylene and fresh 100% xylene for 5 minutes each. Then the tissues were waxed in 50% xylene and 50% paraffin for 1 hour, then immersed in 100% paraffin overnight, and transferred to fresh 100% paraffin for 1 hour the next day. After embedding with an embedding machine, the tissues were frozen at -20°C overnight. The tissue sections were performed by paraffin slicer.

### Step 6: Immunofluorescence Staining

The sections were put sequentially in xylene I for 15 minutes-xylene II for 15 minutes-anhydrous ethanol I for 5 minutes-anhydrous ethanol II for 5 minutes-85% ethanol for 5 minutes-75% ethanol for 5 minutes-distilled water for washing. The sections were placed in a repair box filled with EDTA antigen repair buffer (pH 8.0) in a microwave oven for antigen repair. Heat on medium heat for 8 minutes until boiling, stop the heat for 8 minutes, then turn to medium-low heat for 7 minutes. After natural cooling, the sections were placed in PBS (pH 7.4) and washed on a decolorizing shaker for 3 times, 5 minutes each time. After the sections were slightly dried, circles were drawn around the tissue with a histochemistry pen to prevent antibody from flowing away, PBS was shaken off, BSA was added to block for 30min. The blocking solution was gently shaken off and the primary antibody prepared in a certain proportion of PBS was added on the sections, layed flat in humidified box and incubated at 4°C overnight. The sections were placed in PBS (pH 7.4) and washed on a decolorizing shaker for 3 times, 5 minutes each time. After the sections are slightly dried, the secondary antibody was added to the circle to cover the tissue and incubated in the dark at room temperature for 50 minutes. The sections were placed in PBS (pH 7.4) and washed on a decolorizing shaker for 3 times, 5 minutes each time. After the sections are slightly dried, DAPI staining solution was added to the circle, incubated in the dark at room temperature for 10 minutes. The sections were placed in PBS (pH 7.4) on a decolorizing shaker for 3 times, 5 minutes each time. Autofluorescence quencher was added to the circle for 5 minutes, then rinsed with running water for 10 minutes. After the sections are slightly dried, the sections were sealed with anti-fluorescence quencher sealing medium. The sections were observed and images were collected under a fluorescence microscope (DAPI: UV excitation wavelength 330-380nm, emission wavelength 420nm, blue light; CY3 excitation wavelength 510-560nm, emission wavelength 590nm, red light).

### Experimental Results

As shown in Figure 15, the glucose-regulated probiotic drug factory can be activated in the wounds of diabetic mice to express and secrete human acidic fibroblast growth factor rhaFGF135 and chemokine CXCL12.

### Example 19: Study on the Promotion of Skin Wound Healing in Diabetic Mice by Probiotic Drug Factories Regulating the Expression and Secretion of Human Acidic Fibroblast Growth Factor rhaFGF135 and Chemokine CXCL12

In this example, the skin wounds of type 1 diabetic mice were taken as an example to demonstrate the promotion of the healing of mouse skin wounds by glucose-regulated probiotic drug factories, without limiting the scope of the protection of the present invention. The specific steps are as follows:

### Step 1: Bacterial Culture

The specific steps are the same as in Example 8 of the present invention.

### Step 2: Cell Collection

The recombinant probiotics selected with good induction secretion effects of human acidic fibroblast growth factor rhaFGF135 and chemokine CXCL12 selected in Examples 5-7, and wild-type EcN were amplified and cultured. The culture was centrifuged at 5000 rpm for 5 minutes, washed three times with sterile PBS (pH 6.35), and resuspended to a concentration of 10⁹CFU/10µl.

### Step 3: In Situ Bacteria Drip Application

PBS, human acidic fibroblast growth factor rhaFGF135, wild-type EcN, probiotics suspension regulating the expression and secretion of rhaFGF135, and probiotics suspension regulating the expression and secretion of CXCL12 were respectively dripped in situ onto the skin wounds of type 1 diabetic mice, 10 µL (10⁹ CFU engineered bacteria) per mouse every 24 hours.

### Step 4: Measurement of Skin Wound Size

The skin wounds was photographed every 24 hours, and the wound area was measured using Image J software.

### Experimental Results

As shown in Figure 16, the in situ dripping of probiotics regulating the expression and secretion of rhaFGF135 and CXCL12 significantly promotes the healing of skin wounds in diabetic mice.

### Example 20: Safety Verification of Probiotic Drug Factories in Promoting Skin Wound Healing

### Step 1: Bacterial Culture

The specific steps are the same as in Example 7 of the present invention.

### Step 2: Cell Collection

The specific steps are the same as in Example 4 of the present invention.

### Step 3: In Situ Bacteria Drip Application

The specific steps are the same as in Example 4 of the present invention.

### Step 4: Sampling

24 hours after bacterial application, the blood of mice was collected, and then the mice were killed. The heart, liver, spleen, lung, and kidney were taken respectively, and added with PBS buffer and ground.

### Step 5: Safety Verification

The blood of mice was analyzed for routine blood analysis; the tissue grinding liquid was applied to the LB plate, and the colony count was performed after static culture at 37°C for 12 hours.

### Experimental Results

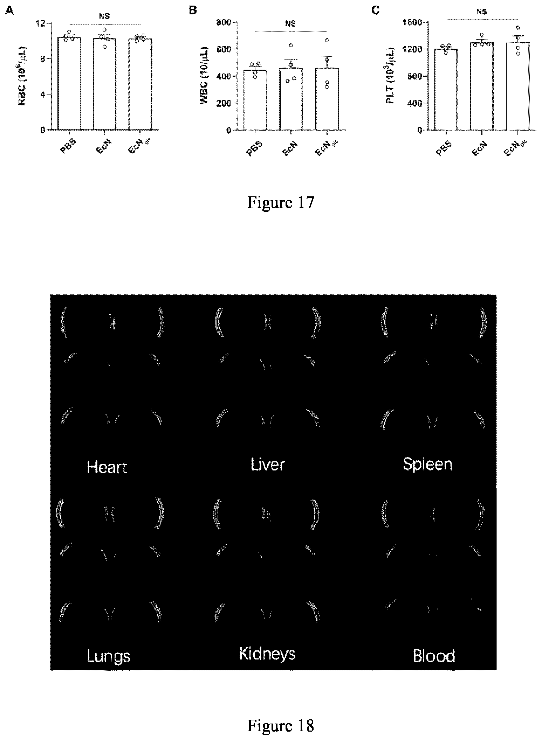

As shown in Figures 17 and 18, the in situ dripping of probiotics does not affect the blood indicators of the mice, and the bacteria does not infect the organs of the mice, and there is no residue in the blood after 24 hours.

The scope of the present invention is not limited to the above examples. Any variations and advantages conceived by those skilled in the art without departing from the spirit and scope of the concept of the invention are all included within the scope of the present invention, protected by the appended claims.

## Claims

1. A glucose-regulated gene expression loop control system, comprising a transcription repressor HexR, a glucose-induced strong promoter, and a sequence to be transcribed.

2. The glucose-regulated gene expression loop control system of claim 1, wherein the transcription repressor HexR can undergo homodimerization and bind to a DNA sequence selected from a group consisting of SEQ ID NO: 78 to SEQ ID NO: 80.

3. The glucose-regulated gene expression loop control system of claim 2, wherein the glucose-induced strong promoter is formed by combining a constitutive strong promoter with the DNA sequence, and the glucose-induced strong promoter comprises a nucleotide sequence selected from a group consisting of SEQ ID NO: 1 to SEQ ID NO: 10.

4. The glucose-regulated gene expression loop control system of claim 1, wherein the transcription repressor HexR can bind to the glucose-induced strong promoter, thereby repressing the expression of the sequence to be transcribed.

5. The glucose-regulated gene expression loop control system of claim 1, wherein the sequence to be transcribed comprises a single coding expression reporter protein or functional protein, or a tandem expression of multiple proteins.

6. The glucose-regulated gene expression loop control system of claim 1, wherein the transcription repressor HexR, the glucose-induced strong promoter, and the sequence to be transcribed can be constructed on one or two plasmid vectors.

7. A glucose-regulated gene expression regulation method, being regulated by the glucose-regulated gene expression loop control system of claim 1.

8. The glucose-regulated gene expression regulation method of claim 7, wherein
when glucose is absent, the transcription repressor can bind to the glucose-induced strong promoter, thereby repressing the expression of the sequence to be transcribed; or
when glucose is present, the glucose is metabolized via an Entner-Doudoroff pathway to generate a metabolic intermediate 2-keto-3-deoxy-6-phosphogluconate (KDPG), which blocks binding between the transcription repressor and the glucose-induced strong promoter, causing the transcription repressor to dissociate from the glucose-induced strong promoter, thereby initiating the expression of the sequence to be transcribed.

9. An expression vector, engineered cell, sensor, or recombinant probiotic, comprising the glucose-regulated gene expression loop control system of claim 1.

10. The expression vector, engineered cell, sensor, or recombinant probiotic of claim 9, wherein the glucose-regulated gene expression loop control system is located in a chromosome or plasmid of the engineered cell, sensor, or recombinant probiotic.

11. The application of glucose-regulated gene expression loop control system of claim 1, the expression vector, engineered cell, sensor, or recombinant probiotic of claim 9 in the preparation of drugs for treating and/or preventing diabetes, and in the preparation of drugs for regulating blood glucose.

12. The application of claim 11, wherein, the application includes oral medications.

13. The application of claim 11, wherein, the application includes regulating blood glucose by controlling the release of GLP-1 and PDX-1 hypoglycemic proteins.

14. A method for regulating blood glucose, using the glucose-regulated gene expression loop control system of claim 1, and/or the expression vector, engineered cell, sensor, or recombinant probiotic of claim 9 to regulate blood glucose.

15. The method of claim 14, wherein the expression of GLP-1 and PDX-1 hypoglycemic proteins is regulated.

16. The method of claim 15, comprising steps of:
a) artificially constructing a prokaryotic expression vector or a functional module for chromosomal integration, wherein the prokaryotic expression vector or functional module for chromosomal integration comprises a glucose-induced GLP-1/PDX-1 expression regulation system;
b) transforming the expression vector or functional module for chromosomal integration constructed in step a) into microbial cells or integrating the expression vector or functional module into chromosome of the microbial cell to prepare engineered cells containing the glucose-induced GLP-1/PDX-1 expression regulation system;
c) administering the engineered cells prepared in step b) by gavage into diabetes model mice; and
d) performing a glucose closed-loop induction to cause the engineered cells to express and secrete GLP-1/PDX-1, which is absorbed into the bloodstream to lower blood glucose levels.

17. The method of claim 14, comprising use of oral probiotics.

18. The glucose-regulated gene expression loop control system of claim 1, the glucose-induced gene expression regulation method of claim 7, the expression vector, engineered cell, sensor, or recombinant probiotic of claim 9, and the method for regulating blood glucose of claim 14, wherein, the glucose-regulated gene expression loop control system has fine-tuning and reversible expression kinetic characteristics; the fine-tuning means that downstream gene expression is precisely regulated by glucose and exhibits a dose-dependent relationship; the reversibility means that the entire process of glucose-regulated gene expression is reversible and can be turned on or off by controlling the presence or absence of glucose.

19. A plasmid, being one or more plasmids selected from a group consisting of pGN11, pGN69, pGN89, pGN90, pGN227, pGN12, pGN228, pGN229, pGN220, pGN221, pGN222, pGN223, pGN224, pGN231, pGN232, pGN233, pGN65, pGN237, pGN238, pGN239, pGN241, pGN242, pGN243, pGN288, pGN308, pGN306, pGN307, and pXG58.

20. A nucleotide sequence, being a glucose-induced strong promoter selected from a group consisting of P_{HexR1}, P_{HexR2}, PHexR3, PHexR4, PHexR5, PHexR6, PHexR7, P_{HexR8}, PHexR9, and P_{hexR10}, and having a nucleotide sequence depicted in SEQ ID NO: 1 to SEQ ID NO: 10.

21. A primer or primer pair, the primer having a DNA sequence selected from a group consisting of SEQ ID NOs: 16-60, 62-65, 67-74, and 76-77; and the primer pair having DNA sequences selected from a group consisting of SEQ ID NOs: 1-30.
